(19)

Europäisches Patentamt
European Patent Office
Office européen des brevets

(11) **EP 2 018 825 A1**

(12) **EUROPEAN PATENT APPLICATION**

(43) Date of publication:
**28.01.2009 Bulletin 2009/05**

(51) Int Cl.:
***A61B 5/11*** (2006.01)

(21) Application number: **07113253.4**

(22) Date of filing: **26.07.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Ecole Polytechnique Fédérale de Lausanne (EPFL)**
**1015 Lausanne (CH)**

(72) Inventors:
• **Ionescu, Anisoara**
**1024, Ecublens (CH)**

• **Aminian, Kamiar**
**1814, La Tour-de-Peilz (CH)**
• **Buchser, Eric**
**1091, Grandvaux (CH)**

(74) Representative: **Grosfillier, Philippe**
**Andre Roland S.A.**
**Intellectual Property Services**
**Avenue Tissot 15**
**P.O. Box 1255**
**1001 Lausanne (CH)**

(54) **Methods and device to quantify human physical activity pattern**

(57)     The method for analyzing physical activity of a subject comprises:

-) measuring at least one of body movement or posture of the subject using at least one sensor;

-) defining a plurality of physical activity time series based on at least one of the measured body movement or posture;

-) characterizing the plurality of physical activity time series based on at least one of fractal analysis or symbolic dynamic statistics to obtain a plurality of non-linear physical activity metrics; and

-) comparing the non-linear physical activity metrics for the subject to non-linear physical activity metrics previously obtained for one or more other subjects.

EP 2 018 825 A1

**Description**

<u>TECHNICAL FIELD</u>

The present invention concerns methods and devices for quantifying human physical activity patterns. More specifically, the invention is aims to obtain an effective tool to quantify human physical activity behavior allowing to objectively assess a chronic disease (pain) state and/or the effect of a medical treatment.

<u>BACKGROUND OF THE INVENTION</u>

It is known in the prior art that quantitative posture, activity and sleep metrics may provide to the clinician an objective indication of different sets of therapy parameters.
In the prior art, there has been no attempt to define physical activity time-series from the basic body posture (lying, sitting, standing, walking) and to analyze the temporal structure of activity pattern with nonlinear methods in order to reveal intrinsic characteristics of human activity in health and disease.

<u>SUMMARY OF THE INVENTION</u>

It is therefore an aim of the invention to improve the known methods and devices.
More specifically, an objective of the invention is to obtain an effective tool to quantify human physical activity behavior allowing to objectively assess a chronic disease (pain) state and/or the effect of a medical treatment.

**An idea of the invention is to:**

- Generate **physical activity** time series from body fixed inertial sensors (accelerometers, gyros)
- Use methods to quantify the temporal structure/pattern of real-life physical activity: such as **(i) fractal analysis (Fano factor analysis, detrended fluctuation analysis), (ii) symbolic dynamic statistics, (iii) cumulative probability distribution.**
- Assess the **chronic pain impact on the patient's daily life activity** by comparing the new defined metrics for chronic pain and healthy control data
- Assess the **effect of pain relief treatment** by comparing the new defined metrics before and after treatment

**Background:** As our understanding of human physical activity is improving, the quantitative approach where activities and patterns can be assessed intuitively (visually) by numbers and graphs, is showing limitations. New paradigms involving nonlinear analysis of less straightforward parameters appear promising. Trials on various biological signals have paved the way for studies looking at the temporal organization of physical activity parameters such as posture changes or walking episodes in patients suffering from chronic pain. Advanced mathematical tools applied to long-term physical activity time series resulted in new metrics and outcome parameters suggesting the presence of predictable correlations between the intensity of pain and features of physical activity.

BRIEF DESCRIPTION OF THE DRAWINGS

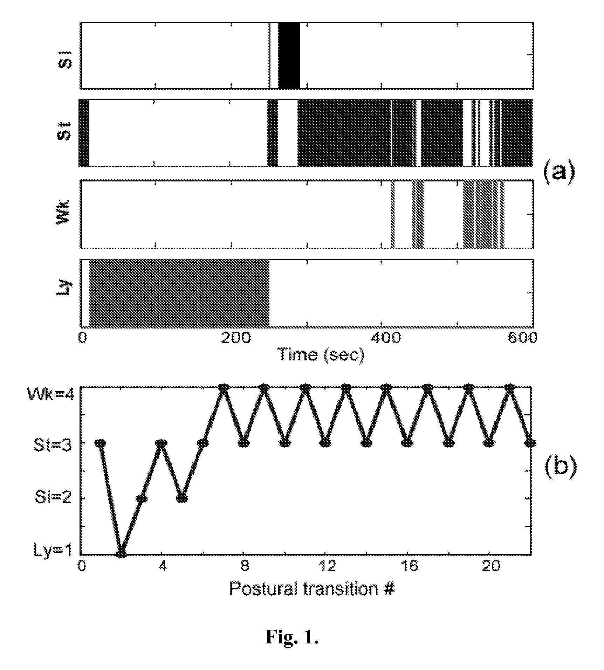

Figure 1 is an illustrative example of posture allocation time series ('*Pattern 1*')

(a) Assessment of postures (sitting-Si, standing-St, lying-Ly) and walking activity (Wk) from body-fixed kinematical sensors,
(b) posture allocation time series obtained from (a); each sample of the time series carry information about the type of the underlying posture/activity, discarding information about the duration;

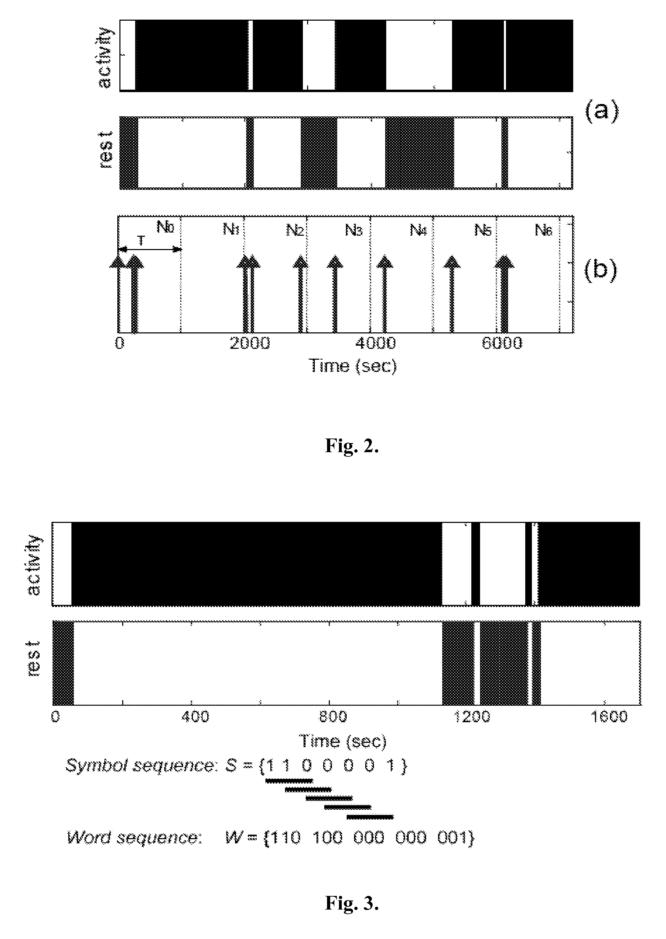

Figure 2 are typical representations activity-rest/rest-activity postural transitions as a stochastic point process ('Pattern 3')

(a) Activity-rest allocation during the monitoring time,
(b) the event series corresponding to the occurrence time of activity-rest/rest-activity postural transitions form a point process. The sequence of counts {Ni} is a discrete-time, nonnegative, integer value stochastic process formed from the point process by recording the number of events in successive counting windows of duration T;

Figure 3 is a symbolic representation of activity-rest allocation ('Pattern 4'). The mapping procedure is based on Eq. 1 ($\varepsilon= 4$) and 3-step template;

Figure 4 is an illustrative example of DFA analysis of posture allocation time-series

(a) Original time-series obtained from five consecutive days recording and surrogate time series generated from a 1st order Markov model with the same transition probability between states as original series,
(b) DFA scaling exponent for original and surrogate data;

Figure 5 is a cumulative probability distribution $P(d)$ of duration of walking episodes ('Pattern2')

(a), (c) Illustrative example of two patterns recorded during five consecutive days in two subjects,
(b) semi-logarithmic plot and double-logarithmic plot (inset) of P(d) of pattern represented in (a);
(d) double-logarithmic plot and semi-logarithmic plot (inset) of P(d) of pattern represented in (c). For the pattern represented in (a) the distribution closely follows a straight line on the semi-logarithmic plot indicating an exponential behaviour while for the pattern represented in (c) the distribution follows a straight line on a double-logarithmic plot indicating a power-law behaviour;

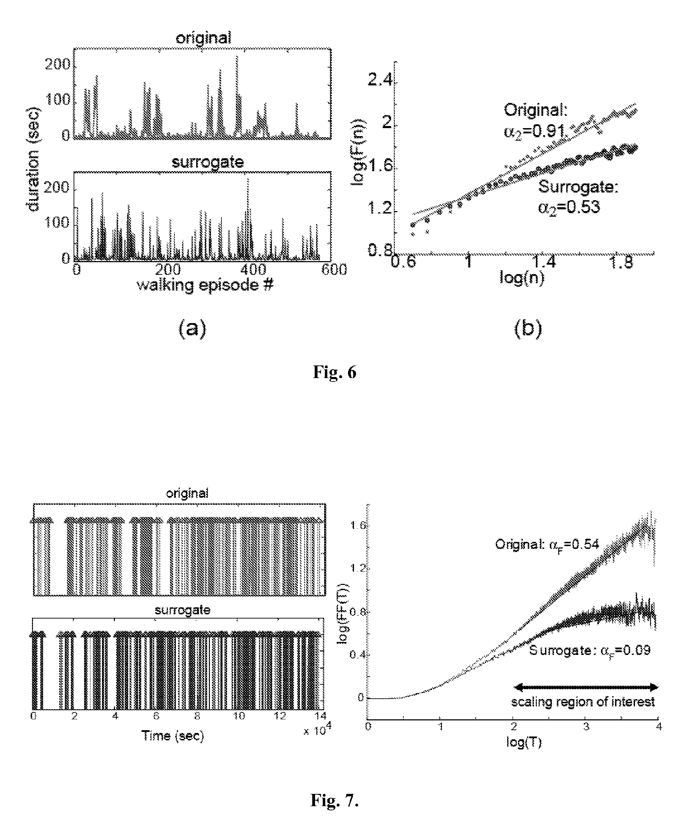

Figure 6 is an Illustrative example of DFA analysis of physical activity time series obtained from the duration of walking episodes.

(a) Original time-series obtained from five consecutive days recording and surrogate time series generated by randomly shuffling the original data
(b) DFA scaling exponent for original and surrogate data;

Figure 7 is an Illustrative example of Fano factor analysis.

(a) Original and surrogate event-series,
(b) double-logarithmic plot of the Fano factor function for a representative healthy subject and the surrogate data obtained by randomly shuffling the interevent intervals. The *scaling region of interest* is for T > 100s where the original data and surrogate differ considerably;

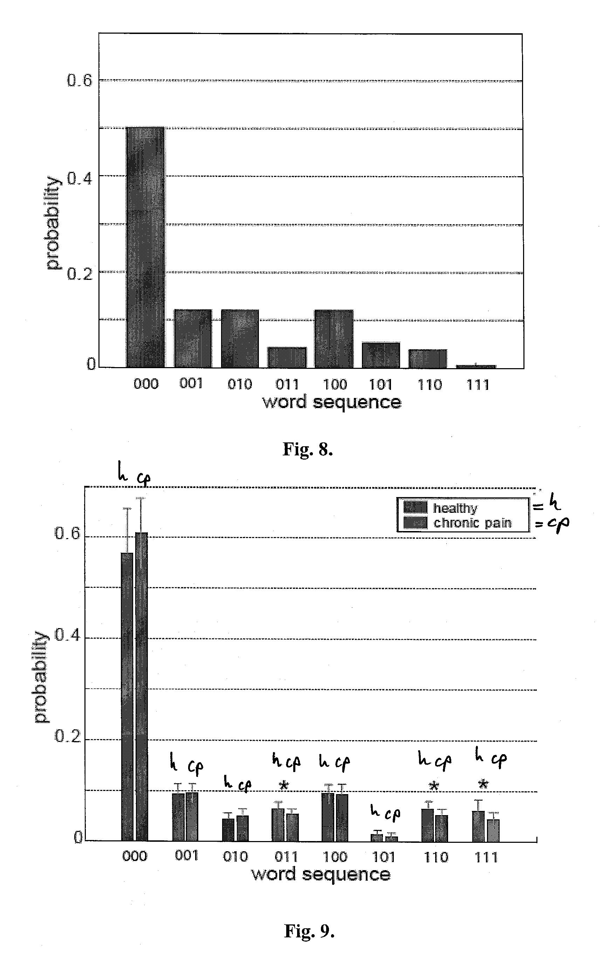

Figure 8 is a word histogram illustrating the probability distribution of the word sequence in a chronic pain patient;

Figure 9 is a Word histogram illustrating the probability distribution (*mean $\pm$ std*) of the word sequence in healthy control and chronic pain groups.

DETAILED DESCRIPTION:

The method according to the invention comprises the following steps:

- Record long term body movements/postures using inertial sensors (accelerometers and gyroscopes) fixed on the body
- Define different ***physical activity time-series*** from recorded body movements/postures, for example:

(i) the sequence of posture allocations quantified from low to high activity, i.e. lying=1, sitting=2, standing=3, walking=4,
(ii) the sequence of daily walking episodes characterized by their duration,
(iii) the occurrence time of activity-rest postural transitions (activity = walking/standing, rest = lying/sitting) treated as a point process, i.e., as a sequence of events distributed on the time axis.

- Quantify/characterize the pattern of physical activity time-series using ***fractal analysis (cumulative distribution function, detrended fluctuation analysis, Fano factor analysis) and symbolic dynamic statistics***.
- Compare the ***nonlinear physical activity metrics*** obtained from healthy control and chronic pain data to see if the metrics are significantly different in chronic pain conditions compared to healthy control.

The dependent method claims define specific steps of the method according to the invention.

In addition, the claims also define a device suitable for carrying out the method according to the invention.

The present invention will be best understood by the following description relating to the physical activity monitoring system and the measurement protocol (II), to defining the different physical activity time-series (III), to presenting mathematical tools and to quantifying the dynamics of temporal activity patterns (IV), to presenting results (V), and to discussing the findings (VI).

II DATA RECORDING

The daily motor function was recorded using an ambulatory system based on three inertial sensors (each composed on 2 accelerometers and 1 gyroscope) fixed on the chest, the thigh and the shank. Given this particular sensor configuration and the use of appropriate software [13] it is possible to detect and analyze a number of physical activity parameters including the sitting, standing and lying posture, transition between postures, walking activity and dynamic gait variables. The monitoring system and the posture analysis algorithms were validated in chronic pain patients treated by spinal cord stimulation (SCS). By considering 61 hours of data obtained from 21 patients before and after SCS, the sensitivity and specificity in detecting lying, sitting, standing and walking were superior to 95% [13]. The system was designed to record physical activity in the normal life environment over prolonged periods of time.

In this study, body posture and movements were recorded in 15 patients (7 female, 8 males, age = $66 \pm 14$) suffering from chronic intractable pain caused by spinal stenosis, peripheral artery disease or polyneuropathy and in 15 age-matched healthy subjects (8 females, 7 males). Measurements of physical activity of the daily living were performed using the ambulatory system. Signals from inertial sensors were recorded at a sampling rate of 40Hz during 5 consecutive days and 8 hours each day. In the chronic pain group pain intensity was assessed on the 10-cm Visual Analogue Scale (VAS).

III. DEFINITION OF PHYSICAL ACTIVITY PATTERNS

Humans participate on a daily basis in a large number of distinct activities ranging from planned events (work, recreation) to unforeseen random events and *spontaneous activity* which is biologically determined as part of a feedback system that homeostatically controls body weight [8], [9]. Factors such as chronic disorders including chronic pain, fatigue or depression can modify the usual course of daily living either because of disease related impairment, constraints to carry out specific tasks or both.

Notwithstanding the complexity of factors controlling human activity, all daily life activities are spent in one or more basic body postures (sitting, standing, lying) including walking. Since physical activity is both very variable and influenced by a myriad of physiological and pathological causes, quantitative parameters only provide a partial assessment and do not allow for a distinction between normal and abnormal activity.

In addition to the measurement of quantitative aspects, a complete assessment of physical activity requires the measurement and analysis of the intricate pattern of postures, movements and transitions between postures.

The sequence, the timing, the time spent in a posture and any combination thereof can be used to define *physical activity time series* that can be analyzed with appropriate mathematical tools to provide information which might be either characteristic of normal physical activity or specific of one or more disorders.

A. Sequence of posture allocation (*Pattern 1*)

By gathering posture parameters over the measurement time (5 consecutive days, 8 hours per day) we consider the sequence of posture allocation quantified from low to high intensity, i.e., lying=1, sitting=2, standing=3, walking=4 (Fig. 1). Each sample of the generated time series corresponds to the type of the underlying posture/activity and discards information about duration, therefore the length of the time series is related to the number of postural changes during the monitoring time.

B. Duration of walking periods (*Pattern 2*)

Many authors consider walking to be the quintessential activity and it is often suggested the measurement of the walking is an adequate representation of a person's overall activity level [14]. [15]. As almost every task in daily routine activities implies the action of walking, we expect the sequence of walking episodes characterized by their duration to provide reliable information about the temporal organization of physical activity in real life conditions.

C. Time of activity-rest transitions as point process (event-series) (*Pattern 3*)

A variety of human actions are initiated by activity-to-rest or rest-to-activity postural transitions. Quantifying the timing of human actions in daily life could be of great importance to understand human behavior in relation with chronic diseases such as chronic pain, chronic fatigue syndrome and neurodegenerative disorders such as Alzheimer's disease.

We have defined *activity* as standing or walking and *rest* as sitting or lying postures. The statistical behavior of activity-rest daily pattern can be studied replacing the complex waveform by *point events* corresponding to the time of transitions between postures. Mathematically, the sequence of postural transitions can be viewed as a realization of a *stochastic point process* specified by the set of occurrence time $\{t_i\}$ of transitions.

The features of the defined point process can be studied in terms of the sequence of numbers of events (counts) $\{N_i\}$. Figure 2 illustrates how the sequence is obtained. The time axis is divided into equally spaced contiguous time windows, each of duration of $T$ seconds, and the number of events in the $i$th window is counted and denoted as $N_i$. This sequence $\{N_i\}$ forms a discrete-time random counting process of non-negative integers. An attractive feature of this representation is that it preserves the correspondence between the discrete time axis of the counting process $\{N_i\}$ and the absolute real axis of the underlying point process.

D. Symbolic sequence from comparison of successive rest-activity-rest periods (*Pattern 4*)

Another way to characterize the sequence of successive activity-rest periods is to use a context dependent *symbolic description*. A coding procedure suitable to assess the dynamics of daily activity in relation with chronic diseases such as lower limbs/low back chronic pain, chronic fatigue syndrome, cardiovascular disease, etc., is to compare the duration of each activity period ($a_j$) with the rest periods just before ($r_{j-1}$) and after it ($r_{j+1}$). In this way, the original daily activity-rest sequence can be represented as a *symbolic sequence, $S = S_1,...,S_k$ ($k$ = 1, $M$-1)*:

$$if \quad a_j \geq \varepsilon r_{j-1} \quad then \quad S_k = 1$$
$$else \quad S_k = 0$$
$$if \quad a_j \geq \varepsilon r_{j+1} \quad then \quad S_{k+1} = 1 \qquad (1)$$
$$else \quad S_{k+1} = 0$$

where $r_{j-1}, \alpha_j, r_{j+1}, j = 1, M$ is the succession of rest-activity-rest periods and $\varepsilon$ is an arbitrary constant.

After symbolization, the next step in identification of temporal patterns is the construction of word sequence W, from the symbol series S by collecting groups of symbols together in temporal order. This sequencing process typically involves definition of a finite-length template (for example 3-step template) that can be moved along the symbol series one step at a time, each step revealing a new sequence (word). Figure 3 illustrates this process for an activity-rest time series that has been initially converted into a binary symbol series based on equation (1). If we look for the occurrence of symbol sequences consisting of 3 consecutive symbols will be 8 ($2^3$) possible distinct words which can be observed.

IV. STRUCTURAL/NONLINEAR ANALYSIS METHODS

A. Fractal analysis

The dynamics of a stochastic series can be explored through its correlation properties, or in other words, the time ordering of the series. *Fractal analysis* is an appropriate method to characterize complex time series by focusing on the time-evolutionary properties of the data series and on their correlation structure. Fractal structures are characterized by complex pattern of correlations appearing following multiple time scales. In such process, the value at a particular time is related not just to the immediately preceding values, but also to fluctuations in the remote past. Fractal time series are also characterized by *self-similarity (scale invariance)*, signifying that the statistical properties of segments within the series are similar, whatever the time-scale of observation. In mathematical terms a time series $y(t)$ is self-similar if $y(t) \equiv a^a y(t/a)$ where $\equiv$ means that the statistical properties of the both sides of equation are identical, a is the change in scale and $a$ is the self-similarity parameter (scaling exponent).

In the context of stochastic point processes, a fractal data set exhibits clusters of events in time. Self-similar clusters have smaller clusters within larger clusters of clusters (burst of events interspersed with quiescent periods at many different time-scales).

To quantify the characteristics of the temporal correlations in the physical activity time-series we use the *cumulative distribution function (CDF)*, *detrended fluctuation analysis* (DFA) and *Fano factor analysis (FFA)* methods.

### 1. Cumulative distribution of durations

The distribution of duration of activities/postures (walking, activity, rest) is a useful measure for studying the underlying dynamics and the fractal properties of long-term recorded activity. In this study we used the cumulative distribution $P(d)$, which is defined as [16]:

$$P(d) \equiv \int_d^\infty p(r)\mathrm{d}r \qquad (2)$$

where $p(r)$ is the probability density function of durations between $r$ and $r + \mathrm{d}r$.

Finding a power-law behaviour for the distribution of duration (activity, rest, walking), $P(d) \propto d^{-\alpha}$, suggests a scale invariant dynamic typical for fractal-like phenomena. The exponential behaviour $P(d) \propto e^{-d/\tau}$ for the distribution of duration suggests a dynamical process with a characteristic time scale, $\tau$.

### 2. Detrended fluctuation analysis

The DFA method can quantify the temporal organization of the fluctuations in a given non-stationary time-series by a single scaling exponent $\alpha$ - a self-similarity parameter that represents the long-range power-law correlation properties of the signal. The scaling exponent $\alpha$ is obtained by computing the root-mean-square fluctuation $F(n)$ of integrated and detrended time series at different observation windows of size $n$ and plotting $F(n)$ against $n$ on a log-log scale. Fractal signals are characterised by a power law relation between the average magnitudes of the fluctuations $F(n)$ and the number of points $n$, $F(n) \sim n^\alpha$. The slope of the regression line relating log $F(n)$ to log $n$ determines the scaling exponent $\alpha$. The time series of fractal signals can therefore be indexed by the deviation of $\alpha$ from 0.5 to 1. For a value of $\alpha = 0.5$ the signal is random; increasing values of $\alpha$ ($0.5 < \alpha \leq 1$) indicate rising power-law scaling behaviour of the signal and the presence of long-range (fractal like) correlations. The scaling range allowing for a correct assessment of the power-law behaviour is $5 \leq n \leq L/10$, where L is the length of the time-series [17].

One important advantage of DFA over conventional methods (e.g., spectral analysis) is that it permits the detection of intrinsic self-similarity embedded in a seemingly nonstationary time series, and also avoids the spurious detection of apparent self-similarity, which may be an artefact of extrinsic trends. This method has been successfully applied to a wide range of simulated and physiologic time series in recent years [18], [19], [20].

### 3. Fano Factor analysis

*Fano factor (FF)* is a useful statistical measure to test whether the fluctuations of activity-rest/rest-activity transitions counts $\{N_i\}$ illustrated in Fig. 2 occur randomly or are of a time-scale invariant (fractal) nature. The Fano factor $FF(T)$ is defined as the event-number variance divided by the event-number mean, which is a function of the counting time T (sec):

$$FF(T) = \frac{\left\langle N_i^2(T) \right\rangle - \left\langle N_i(T) \right\rangle^2}{\left\langle N_i(T) \right\rangle} \qquad (3)$$

where < > denotes the expectation value and $N_i(T)$ is the number of transitions in the $i^{th}$ window of length T [21], [22].

The Fano factor curve is constructed by plotting $FF(T)$ as a function of the window size T on a log-log scale. For a data block of length $T_{max}$, T is progressively increased from a minimum of 2 sec to a maximum of $T_{max}/10$ so that at least ten non-overlapping windows are used for each measure of $FF(T)$. For a random Poisson process $FF(T)$ is approximately 1.0 for all window sizes [22], [23]. For a periodic process $FF(T)$ approaches zero as the window size increases due to decreasing variance. For a *fractal-rate stochastic point process* (the fractal character lie in the dependencies among the interevent intervals) the $FF(T)$ assumes a power-law form for large T :

$$FF(T) = 1 + \left(\frac{T}{T_0}\right)^{\alpha_F}, 0 < \alpha_F < 1 \qquad (4)$$

where $T_0$ is the fractal onset time, and marks the lower limit for significant scaling behaviour in the *FF*. The power-law relationship appears as a straight line on the log-log scale with a positive slope $\alpha_F$. The parameter $\alpha_F$ is defined as the fractal scaling exponent of the point process. The correlation coefficient is used to test for linearity on the log-log scale, and linear regression is used to calculate $\alpha_F$.

The *FF* indicates the degree of event-clustering (*FF*>1) or anti-clustering (*FF*<1) in a point process relative to the standard homogeneous Poisson process for which *FF(T)*= 1 for all *T*.

Whether or not a power law relationship in the *FF* curve reflects a fractal rate process (characterized by clusters of events at many time-scales) is tested by constructing *surrogate data* sets in which the inter event intervals (duration of activity and rest periods) are randomly shuffled. Specifically, we randomly shuffled the activity and the rest periods separately then constructed the surrogate time-series by altering the shuffled activity and rest periods. This creates a randomized data set with activity and rest periods (duration) identical to the original time-series but with randomized activity-rest and rest-activity transition times. The lack of fractal behavior in the FF of the shuffled data suggests that the power law features are related to the ordering of activity and rest periods in the original data.

U. Fano first used this method for characterising statistically the fluctuation in the number of ions generated by fast charged particles. In physiological systems, Fano factor analysis has been used to demonstrate the fractal characteristics of heart rate variability, and the firing patterns of sympathetic, auditory and visual neurons [22], [23], [24], [25].

### B. Symbolic dynamics statistics

The temporal structure of the symbolic sequences defined in Fig. 3 is revealed by the probability of each possible symbol sequence obtained by counting the number of times each word occurs ($nw_k$) and dividing by the total number of observed words *(NW): $P_{wk} = nw_k/NW$*.

Another useful measure is the *transition probability matrix* (*T*) between different words with elements $T_{wiwj}$, defined as $T_{wiwj} = N_{wiwj}/NT$, where $N_{wiwj}$, is the number of transitions from word $w_i$ to word $w_j$ during the monitoring time and *NT* is the total number of transitions.

### C. Statistical Analysis

In order to study the sensitivity of each method to differentiate chronic pain behavior from healthy control, we have calculated the parameters that quantify the temporal dynamics of the physical activity pattern (scaling exponents, symbolic dynamics statistics) for each subject as well as the means and the standard deviations in both healthy control and chronic pain groups. Differences between the two groups were assessed using the two-sided Mann-Whitney test.

### V. RESULTS

### A. Pattern 1 - DFA

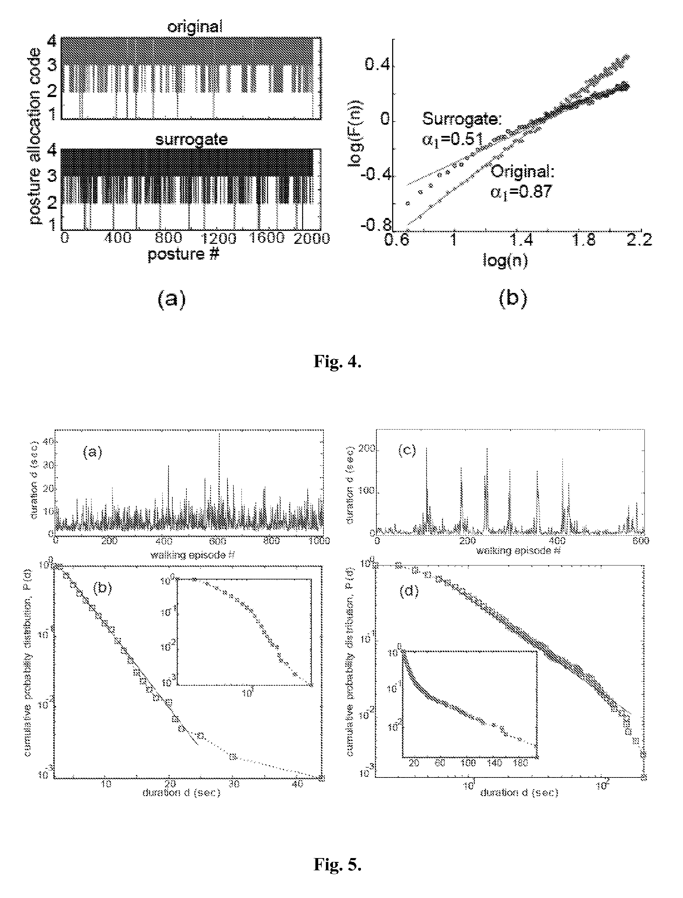

The sequence of posture allocation in Fig. 1 was analyzed using the DFA method. Figure 4a (top) shows a typical example of postures and activities sequences from five consecutive daily recordings in one subject (starting at about the same time in the morning). An intrinsic property of this original time series is the order of the data points. As the quantitative nature of the temporal organization of these time series is intuitively unremarkable, we applied the DFA method to detect potential long-range correlations. The validity of the results is verified by considering *surrogate sequences* with known statistical properties [29]. The most straightforward way in this case is to build a 1st order Markov process having the same transition probabilities as the original sequence but with data points in a shuffled order (Fig. 4a-bottom). As illustrated in Fig. 4b, the fluctuations in the original time series exhibit long-range correlations ($\alpha_1 = 0.87$), whereas the surrogate data behave as uncorrelated white noise ($\alpha_1 = 0.5$).

### B. Pattern 2 - CDF, DFA

The pattern emerging from the analysis of the duration of walking episodes can display very different statistical properties, ranging from exponential (Figs. 5a, 5b) to scale free power law distribution (Figs. 5c, 5d). This suggests that for a given clinical outcome the appropriate analysis may involve different methods.

The power law form of CDF indicates that there are a large number of short episodes, a smaller number of medium and a few long episodes. If the power law form extends over at least two orders in time then: *(i)* such data cannot be meaningfully characterized by mean and variance, though *(ii)* a proper assessment is provided by the slope of power law CDF and the self-similar correlations in the fluctuations of the data, and *(iii)* the respective patterns may result from sequences of indoor vs outdoor walking, since continuous walking during hundred of seconds is more likely to occur outdoor.

Fig. 6a (top) shows an example of time series obtained from the duration of walking episodes recorded during five consecutive days. DFA analysis (Figs. 6b) reveals the presence of long-range correlations in the fluctuations of walking episodes ($\alpha_2$ = 0.76) suggesting that under normal conditions, the walking episodes have a duration that is organized rather than random. This hypothesis is confirmed by the analysis of surrogate data (obtained by randomly shuffling the samples of the original time series - Fig. 6a-bottom) that show no long-range correlations ($\alpha_2$ = 0.53).

C. Pattern 3- FF

An illustrative example of FF method for original and surrogate data is shown in Fig. 7. The Fano factor curve for the original event series of time of activity-rest transitions deviates from those of surrogate data. The slope of the power law in the Fano factor curve for the original data is $\alpha_F$ = 0.54. The monotonic power-law increase indicates the presence of fluctuations on many time-scales, with the exponent $\alpha_F$ identified as an estimate of the fractal exponent of the point process describing the original *'pattern* 3'. However, a plot of the FF alone cannot reveal whether this large Fano factor arise from the distribution of the interevent intervals, or from their ordering. This issue is resolved by plotting the Fano factor for the shuffled intervals. The FF constructed from the shuffled data retains information about the relative size of intervals only; the shuffling process destroys all correlations and dependencies among the intervals.

D. Pattern 4-symbolic dynamics statistics

Figure 8 illustrates the word frequency distribution in a chronic pain patient. The mapping from daily activity-rest allocation to symbols is based on relation (1) with $\varepsilon = 4$. The picture emerging from this analysis is that the probability of words related to successive '*long activity, short rest*', that is '111', '110' and '011' is lower than the probability of words related to successive '*short activity, long rest*' i.e. '000', '001', '010', '100', '101'.

E. Clinical significance

For the chronic pain group the average pain intensity and its standard deviation on a 1 0cm-VAS was $6\pm2$. The healthy control group was pain free.
In both groups, DFA showed fractal time-structure in the daily posture allocation (*pattern 1*) though the average the scaling exponent ($\alpha_1$) was significantly smaller in chronic pain patients ($\alpha_1$ = 0.756$\pm$0.090) than in control $\alpha_1$ = 0.856$\pm$0.096 - Table I). It follows that in chronic pain patients, some aspects of daily life activity have lost the temporal organization that is found under normal healthy conditions.
Previous studies have suggested that factors such as pain, fear of pain or depression can decrease the physical activity of chronic pain patients though it appears that this is not always the case [30]. It should be pointed out that until now, physical activity has always been assessed either by questionnaires and other similar tools based on self reporting, or by devices measuring quantitative parameters such as stride length, walking distance or even speed. While it is largely recognized that self-reporting is unreliable, the interpretation of quantitative parameters is difficult because factors unrelated to the intensity of pain, whether physiological (age) or pathological (cardio-respiratory or orthopedic disorders, etc) may have a profound influence on physical activity. The nonlinear analysis of physical activity patterns may significantly improve our ability to distinguish between pain and non-pain related behaviors though it has been shown that age as well as various medical disorders can change the organization of a number of biological signals.
In patients with chronic lower limb or low back pain, walking is often impaired and changes in walking patterns may therefore be very important. The analysis of the fluctuations of the correlations of the duration of walking episodes *(pattern2)* showed that the scaling exponent is significantly smaller in the chronic pain group ($\alpha_2$ = 0.734$\pm$0.100 vs. $\alpha_2$ = 0.800$\pm$0.076 - Table I). In addition, the distribution of the walking episodes $P(d)$ was different. With healthy subjects (control group) an exponential, scale free distribution $P(d)$ was found in 11 out of 15 subjects. On the other hand, in only 6 out of 15 chronic pain patients did the distribution P(d) show a power law decay.
The *FF* analysis revealed that under normal healthy conditions the timing of activity-to-rest transitions (*pattern3*) follows a power-law distribution ($0<\alpha_F\leq1$) suggesting time-clustering of activities at different time scales (ranging from ~2min to 4 hours). Statistical analysis (Table I) showed significantly higher value of $\alpha_F$ for healthy controls ($\alpha_F$= 0.350$\pm$0.085) than for chronic pain patients ($\alpha_F$= 0.178$\pm$0.100), suggesting that activity-to-rest transitions are randomly spread over time with pain patients as opposed to organized in healthy people. Even though a large variety of factors can affect activities of daily life that are initiated by activity-to-rest transitions, the bursty feature that we observed seems to be robust. Although the significance, if any, of this finding is unclear and obviously requires specifically dedicated research it may relate to recent studies who suggest that the bursty nature of human behavior is a consequence of a decision-based queuing process where tasks are executed on the basis of their perceived priority. In this concept, most tasks are executed quickly whereas a few are delayed for long period of time [31], [32], [33].
Figure 9 shows the mean value of the probability of words for the two groups, with an arbitrary value of $\varepsilon$ relation

(1). The words '000', '001' (*short activity, long rest*) are more frequent in the chronic pain group while '111', '110', '011' *(long activity short rest)* are more frequent in the healthy control group. We believe this to be related to either pain or fatigue or both that intuitively are likely to be more frequent in pain patients than in healthy subjects.

Statistical analysis shows that the probability of words '111', '110' and '011' is significantly greater for the healthy control than for the chronic pain group (Table II). Moreover, the probability of transition from '1111' to '111' is significantly greater in healthy controls ($0.540 \pm 0.08$) than in chronic pain patients ($0.400 \pm 0.06$).

The above results suggest that the parameters quantifying the temporal structure of physical activity pattern locate significant difference between healthy control and chronic pain groups.

## VI. DISCUSSION

The advances in sensor technology have been associated with substantial improvements in the quality and the reliability of the data. Though better system have allowed long-term recording of quantitative parameters (distances, speed) as well as postures (lying, sitting, standing, walking), the conventional analysis proved unable to reliably discriminate between physical activity in health and disease.

A new approach to analyze human activity is proposed by the present invention. Using nonlinear methods derived from statistical physics (fractal analysis, symbolic dynamics) the temporal organization of physical activity is assessed from time-series (patterns) of defined events. The preliminary results that are presented demonstrate that healthy subjects and chronic pain patients can be discriminated reliably on the basis of specific patterns of their physical activity. Subtle though obvious clinical changes may therefore be quantified which may significantly improve the assessment of symptomatic treatments and perhaps even diagnostic accuracy.

*Fractal analysis* has shown that scale invariance appears to be as a general mechanism underlying many physiological functions [34], [35]. While physical activity and energy consumption are directly linked by obvious mechanisms, their progressive decrease with age is less clear although a "biological control center" has been postulated [8], [9]. Similarly, we have an intuitive understanding of why physical activity is reduced during a painful or incapacitating disease but the notion of a fundamental pattern in physical activity does not refer to common physiological knowledge. Yet, our findings suggest that physical activity in normal subjects has a fractal structure and that this organization is disrupted by chronic pain. The reason as to why there is a fractal pattern and what its clinical (or physiological) significance may be is unclear, and requires specifically dedicated research.

The number of data points needed for fractal analysis is crucial because finite size effects can occur with short time series. As the time series obtained from physical activity monitoring during one day are short (especially for chronic pain patients), we applied DFA and FFA on time series obtained by stitching together data from the five consecutive days. With this approach the resulting time series are characterized by four discontinuities corresponding to the transitions between the monitoring days (physical activity is not recorded from about 17h p.m. to 9h a.m., including the night). Discontinuities are frequent in 'real' data and can arise from the nature of experimental recordings or from the fact that noisy and unreliable portions of continuous physiological recordings must be discarded prior to analysis. Several studies were dedicated to the analysis of this type of nonstationarity on the accuracy of estimated DFA scaling coefficients [26], [27], [28]. It was found that removing segment from a signal and stitching together the remaining parts does not affect the scaling behavior of positively correlated signals even when up to 50% of the points in these signals are removed.

*Symbolic dynamics* appears to be a useful and flexible method to quantify context-depend aspects of activity patterns. The definition of symbols (see equation 1), the word length and the word statistics allows quantifying various aspects of daily posture and activities. In this study we applied the symbolic dynamics method to find out whether episodes of activity are followed by longer resting episodes in patients with presumably increased fatigue or pain or both. The findings indicate that the word frequency and the probability of transition between words are statistically different in pain patients compared to normal controls.

Table I. *Fractal analysis:* Estimated scaling exponents (mean$\pm$std) in healthy control subjects and chronic pain patients.

| Scaling exponents | Healthy subjects | Chronic pain patients (VAS = $6\pm2$) | p-value (Mann-Whitney) |
|---|---|---|---|
| DFA-posture allocation ($\alpha_1$) | $0.856 \pm 0.096$ | $0.756 \pm 0.09$ | p = 0.013 |
| DFA-walking episodes ($\alpha_2$) | $0.800 \pm 0.076$ | $0.734 \pm 0.1$ | p = 0.039 |
| FFA- activity-rest transitions ($\alpha_F$) | $0.350 \pm 0.085$ | $0.178 \pm 0.1$ | p = 0.0002 |

Table II. *Symbol dynamics statistics:* Probability of words (mean±std) in healthy control subjects and chronic pain patients.

| Probability of words | Healthy subjects | Chronic pain patients | p-value |
|---|---|---|---|
| $P_{111}$ | 0.060 ± 0.02 | 0.043 ± 0.01 | p = 0.02 |
| $P_{110}$ | 0.063 ± 0.01 | 0.052 ± 0.01 | p = 0.008 |
| $P_{011}$ | 0.065 ± 0.01 | 0.053 ± 0.01 | p = 0.009 |

**References**

[1] K.F. Janz, Physical activity in epidemiology: moving from questionnaire to objective measurement, British Journal of Sports Medicine, 40, 191 (2006).

[2] K. Aminian, B. Najafi, Capturing human motion using body fixed sensors: outdoor measurement and clinical applications, Comp. Anim. Virtual Worlds, 15, 79, (2004).

[3] E. Buchser, A. Paraschiv-Ionescu, A. Durrer, B. Depierraz, K. Aminian, B. Najafi, B. Rutschmann, Improved mobility in patients treated for chronic pain by spinal cord stimulation, Neuromodulation 8(1), 40, (2005).

[4] K.E. Powell, P.D. Thompson, C.J. Caspersen, J.S. Kendrick, Physical Activity and the Incidence of Coronary Heart Disease, Ann. Rev. Public Health, 8, 253, (1987).

**[5]** J.A. Levine, L.M. Lanningham-Foster, S.K. McCrady, A.C. Krizan, L.R. Olson, P.H. Kane, M.D. Jensen, M.M. Clark, Interindividual Variation in Posture Allocation: Possible Role in Human Obesity, Science, 307(5709), 584, (2005).

[6] K. Westerterp, Pattern and intensity of physical activity, Nature, 410, 539 (2001).

[7] R.C. Martin-Du Pan, R. Benoit, L. Girardier, The role of body position and gravity in the symptoms and treatment of various medical diseases, Swiss Med WKLY, 134, 543, (2004).

[8] T.W. Rowland, The biological basis of physical activity. Med Sci Sports Exerc 1998, 30, 392, (1998).

[9] A. W. Thorburn, J. Proietto, Biological determinants of spontaneous physical activity, Obesity reviews, 1, 87, (2000).

[10] S. P. van der Werf, J.B. Prins, J.H.M.M. Vercoulen, J. W.M. van der Meer, G. Bleijenberg, Indentifying physical activity patterns in chronic fatigue syndrome using the actigraphic assessment, Journal of Psychosomatic Research, 49, 373 (2000).

[11] A. Paraschiv-Ionescu, E. Buchser, B. Rutschmann, S. Coderay, K. Aminian. Long-term monitoring of physical activity pattern in pain patients, Proc. of the 3rd IASTED International Conference on Biomedical Engineering (BIOMED'2005), 614-618, (2005).

[12] A.L. Goldberger, L.A.N. Amaral, J. M. Hausdorff, P.Ch. Ivanov, C.-K. Peng, H.E. Stanley, Fractal dynamics in physiology: Alterations with disease and aging, PNAS, vol. 99 (1), 2466-2472, (2002).

[13] A. Paraschiv-Ionescu, E. Buchser, B. Rutschmann, B. Najafi, K. Aminian, Ambulatory system for the quantitative and qualitative analysis of gait and posture in chronic pain patients treated with spinal cord stimulation. Gait and Posture 20(2), 113, (2004).

[14] D.J. Sieminski, L.L. Cowell, P.S. Montgomery, S.B. Pillai, Physical activity monitoring in patients with peripheral arterial occlusive disease, Journal of Cardiopulmonary Rehabilitation, 17(1), 43-47, (1997).

[15] D.A. Boone, K.L. Coleman, Use of step activity monitor in determining outcomes, Journal of Prosthetics and Orthotics, 18(1S), 86-92, (2006).

[16] J.L. Devore, Probability and Statistics for Engineering and the Sciences, California Polytechnic State University, San Luis Obispo (2000).

[17] C.-K. Peng, S. Havlin, H.E. Stanley. A.L. Goldberger, Quantification of scaling exponents and crossover phenomena in nonstationary heartbeat time series, Chaos, 5(1), 82, (1995).

[18] J.M. Hausdorff, A. Lertratanakul, M. Cudkowicz, A.L. Peterson, D. Kaliton, A.L. Goldberger, dynamic markers of altered gait rhythm in amyotrophic lateral sclerosis, J. Appl. Physiol, 88, 2045, (2000).

[19] K. Hu, P.Ch. Ivanov, Z. Chen, M.F. Hilton, H.E. Stanley, S.A. Shea, Non-random fluctuations and multi-scale dynamics regulation of human activity, Physica A, 337, 307, (2004).

[20] C.-K. Peng, J.E. Mietus, Y. Liu, C.Lee, J.M. Hausdorff, H.E .Stanley, A.L. Goldberger, L.A. Lipsitz, Quantifying Fractal Dynamics of Human Respiration: Age and Gender Effects, Ann. Biomedical Engeneering, 30(5), 683, (2002).

[21] S. Thurner, S.B. Lowen, M.C. Feurstein, C. Heneghan, H.G. Feichtinger, M.C. Teich, Analysis, synthesis, and estimation of fractal-rate stochastic point processes, Fractals 5, 565, (1997).

[22] R.G. Turcott, M.C. Teich, Fractal character of the electrocardiogram: distinguishing heart-failure and normal patients. Ann. Biomed. Eng. 24, 269, (1996).

[23] G.M. Viswanathan, C.-K. Penk, H.E. Stanley, A.L. Goldberger, Deviations from uniform power law scaling in nonstationary time series. Phys. Rev. E, 55, 845, (1997).

[24] M.C. Teich, C. Henehan, S.B. Lowen, T. Ozaki, E. Kaplan, Fractal character of the neural spike train in the visual system in the cat, J. Opt. Soc. Am. 14, 529, (1997).

[25] S.B. Lowen, M.C. Teich,. Auditory-nerve action potentials form a nonrenewal point process over short as well as long time scales. J. Acoust. Soc. Am. 92, 803, (1992).

[26] Z. Chen, P.Ch. Ivanov, K. Hu, H.E. Stanley, Effect of nonstationarities on detrended fluctuation analysis, Physical Review E, 65, 041107, (2002).

[27] J.C. Echeverria, B.R. Hayes-Gill, J.A. Grove, M.S. Woolfson, G.D.H. Croaker, Detrended fluctuation analysis: a suitable method for studying fetal heart rate variability?, Physiological Measurement, 25, 763-774, (2003).

[28] P.S. Wilson, A.C. Tomsett, R. Toumi, Long-memory analysis of time series with missing values, Physical Review E, 68, 017103, (2003)

[29] J. Theiler, S. Eubank, A. Longtin, B. Galdrikian, J.D. Farmer, Testing for nonlinearity in time series: the method of surrogate data, Physica D 58, 77, (1992).

[30] A.A. Vendrig, R. Lousberg, Within-person relationship among pain intensity, mood and physical activity in chronic pain: a naturalistic approach, Pain, 73(1), 71-76, (1997).

[31] A.L. Barabási, The origin of burst and heavy tails in human dynamics, Nature, 435, 207, (2005).

[32] R.V.R. Pandya, Generalized model for human dynamics, e-print physics/0512086, (2005).

[33] A. Vázquez, J.G. Oliveira, Z. Dezsö, K.-II Goh, I. Kondor, A.L Barabási, Modelling bursts and heavy tail in human dynamics, Physical Rev. E, 73, 036127, (2006).

[34] B. Bassingthwaighte, L. Liebovitch, B.J. West, Fractal Physiology, Oxford University Press, 1994.

[35] A.L. Goldberger, L.A.N. Amaral, J.M. Hausdorff, P.Ch. Ivanov, C.-K. Peng, H.E. Stanley, Fractal dynamics in physiology: Alterations with disease and aging, PNAS, 99(1), 2466, (2002).

**Claims**

1.  A method for analyzing physical activity of a subject, the method comprising:

    - ) measuring at least one of body movement or posture of the subject using at least one sensor;
    - ) defining a plurality of physical activity time series based on at least one of the measured body movement or posture;
    - ) characterizing the plurality of physical activity time series based on at least one of fractal analysis or symbolic dynamic statistics to obtain a plurality of non-linear physical activity metrics; and
    - ) comparing the non-linear physical activity metrics for the subject to non-linear physical activity metrics previously obtained for one or more other subjects.

2.  A method as defined in claim 1, wherein the physical activity time series comprises a sequence of posture allocations quantified from low to high activity, respectively lying=1, sitting=2, standing=3 and walking=4, whereby each sample of the generated time series corresponds to the type of posture so that the length of the time series is related to the number of postural changes during a monitoring time.

3.  A method as defined in claim 1, wherein the physical activity time series comprises the duration of successive walking episodes, so that the length of the time series is related to the number of walking episodes recorded during a monitoring time;

4.  A method as defined in claim 1, wherein the physical activity time series comprises the occurrence time of transitions between body rest (sitting, lying) and body activity (walking, standing) modeled as a point process or event series;

5.  A method as defined in one of the preceding claims, wherein complex features embedded in the patterns of physical activity time series are revealed/quantified using fractal analysis comprising cumulative distribution of durations (CDF) or detrended fluctuation analysis (DFA) or Fano Factor analysis (FFA).

6.  A method as defined in claim 1, wherein the physical activity time series comprises a sequence of binary symbols obtained by comparison of successive periods of body rest (sitting, lying) and body activity (walking, standing).

7.  A method as defined in claims 1 and 6, wherein the symbolic dynamics statistics comprise the probability of symbols and the transition probability between symbols.

8.  A device comprising:

    - at least one sensor that measures at least one of body movement or posture of a subject;
    - an analyzer that defines a plurality of physical activity time series based on at least one of the measured body movement or posture, characterizes the plurality of physical activity time series based on at least one of fractal analysis or symbolic dynamic statistics to obtain a plurality of non-linear physical activity metrics, and compares the non-linear physical activity metrics for the subject to non-linear physical activity metrics previously obtained for one or more other subjects.

9.  The device as claimed in claim 8, wherein said sensing means comprise sensors to be fixed on the chest, the thigh and the shank of the subject.

10. The device as claimed in claim 9, wherein each sensor comprises two accelerometers and one gyroscope.

11. The device as claimed in claim 8, 9 or 10, wherein said treatment means comprise at least a computer.

Fig. 1.

**Fig. 2.**

**Fig. 3.**

Fig. 4.

Fig. 5.

Fig. 6

Fig. 7.

**Fig. 8.**

**Fig. 9.**

European Patent Office

**EUROPEAN SEARCH REPORT**

Application Number

EP 07 11 3253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/240086 A1 (AKAY METIN [US]) 27 October 2005 (2005-10-27) * paragraphs [0005], [0025], [0034] - [0042], [0051] * ----- | 1,4,8-11 | INV. A61B5/11 |
| X | NUNES AMARAL L A ET AL: "Power law temporal auto-correlations in day-long records of human physical activity and their alteration with disease" EUROPHYSICS LETTERS, vol. 66(3), 2004, pages 448-454, XP008085517 * the whole document * ----- | 1,5,8-11 | |
| X | OHASHI K ET AL: "Decreased Fractal Correlation in Diurnal Physical Activity in Chronic Fatigue Syndrome" METHODS INF MED, vol. 43, 2004, pages 26-29, XP008085516 * abstract * ----- | 1,5,8-11 | |
| A | PARASCHIV-IONESCU A ET AL: "Ambulatory system for the quantitative and qualitative analysis of gait and posture in chronic pain patients treated with spinal cord stimulation" GAIT & POSTURE, ELSEVIER, vol. 20, no. 2, October 2004 (2004-10), pages 113-125, XP004641647 ISSN: 0966-6362 * the whole document * ----- | 1-11 | TECHNICAL FIELDS SEARCHED (IPC)  A61B |

The present search report has been drawn up for all claims

| Place of search | Date of completion of the search | Examiner |
|---|---|---|
| Berlin | 2 November 2007 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 11 3253

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2007

| Patent document cited in search report | Publication date | Patent family member(s) | Publication date |
|---|---|---|---|
| US 2005240086 A1 | 27-10-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K.F. JANZ.** Physical activity in epidemiology: moving from questionnaire to objective measurement. *British Journal of Sports Medicine,* 2006, vol. 40, 191
- **K. AMINIAN ; B. NAJAFI.** Capturing human motion using body fixed sensors: outdoor measurement and clinical applications. *Comp. Anim. Virtual Worlds,* 2004, vol. 15, 79
- **E. BUCHSER ; A. PARASCHIV-LONESCU ; A. DURRER ; B. DEPIERRAZ ; K. AMINIAN ; B. NAJAFI ; B. RUTSCHMANN.** Improved mobility in patients treated for chronic pain by spinal cord stimulation. *Neuromodulation,* 2005, vol. 8 (1), 40
- **K.E. POWELL ; P.D. THOMPSON ; C.J. CASPERSEN ; J.S. KENDRICK.** Physical Activity and the Incidence of Coronary Heart Disease. *Ann. Rev. Public Health,* 1987, vol. 8, 253
- **J.A. LEVINE ; L.M. LANNINGHAM-FOSTER ; S.K. MCCRADY ; A.C. KRIZAN ; L.R. OLSON ; P.H. KANE ; M.D. JENSEN ; M.M. CLARK.** Interindividual Variation in Posture Allocation: Possible Role in Human Obesity. *Science,* 2005, vol. 307 (5709), 584
- **K. WESTERTERP.** Pattern and intensity of physical activity. *Nature,* 2001, vol. 410, 539
- **R.C. MARTIN-DU PAN ; R. BENOIT ; L. GIRARDIER.** The role of body position and gravity in the symptoms and treatment of various medical diseases. *Swiss Med WKLY,* 2004, vol. 134, 543
- **T.W. ROWLAND.** The biological basis of physical activity. *Med Sci Sports Exerc,* 1998, vol. 30, 392
- **A. W. THORBURN ; J. PROIETTO.** Biological determinants of spontaneous physical activity. *Obesity reviews,* 2000, vol. 1, 87
- **S. P. VAN DER WERF ; J.B. PRINS ; J.H.M.M. VERCOULEN ; J. W.M. VAN DER MEER ; G. BLEIJENBERG.** Indentifying physical activity patterns in chronic fatigue syndrome using the actigraphic assessment. *Journal of Psychosomatic Research,* 2000, vol. 49, 373
- **A. PARASCHIV-LONESCU ; E. BUCHSER ; B. RUTSCHMANN ; S. CODERAY ; K. AMINIAN.** Long-term monitoring of physical activity pattern in pain patients. *Proc. of the 3rd IASTED International Conference on Biomedical Engineering (BIOMED'2005),* 2005, 614-618

- **A.L. GOLDBERGER ; L.A.N. AMARAL ; J. M. HAUSDORFF ; P.CH. IVANOV ; C.-K. PENG ; H.E. STANLEY.** Fractal dynamics in physiology: Alterations with disease and aging. *PNAS,* 2002, vol. 99 (1), 2466-2472
- **A. PARASCHIV-LONESCU ; E. BUCHSER ; B. RUTSCHMANN ; B. NAJAFI ; K. AMINIAN.** Ambulatory system for the quantitative and qualitative analysis of gait and posture in chronic pain patients treated with spinal cord stimulation. *Gait and Posture,* 2004, vol. 20 (2), 113
- **D.J. SIEMINSKI ; L.L. COWELL ; P.S. MONTGOMERY ; S.B. PILLAI.** Physical activity monitoring in patients with peripheral arterial occlusive disease. *Journal of Cardiopulmonary Rehabilitation,* 1997, vol. 17 (1), 43-47
- **D.A. BOONE ; K.L. COLEMAN.** Use of step activity monitor in determining outcomes. *Journal of Prosthetics and Orthotics,* 2006, vol. 18 (1S), 86-92
- **J.L. DEVORE.** Probability and Statistics for Engineering and the Sciences. *California Polytechnic State University, San Luis Obispo,* 2000
- **C.-K. PENG ; S. HAVLIN ; H.E. STANLEY ; A.L. GOLDBERGER.** Quantification of scaling exponents and crossover phenomena in nonstationary heartbeat time series. *Chaos,* 1995, vol. 5 (1), 82
- **J.M. HAUSDORFF ; A. LERTRATANAKUL ; M. CUDKOWICZ ; A.L. PETERSON ; D. KALITON ; A.L. GOLDBERGER.** dynamic markers of altered gait rhythm in amyotrophic lateral sclerosis. *J. Appl. Physiol,* 2000, vol. 88, 2045
- **K. HU ; P.CH. IVANOV ; Z. CHEN ; M.F. HILTON ; H.E. STANLEY ; S.A. SHEA.** Non-random fluctuations and multi-scale dynamics regulation of human activity. *Physica A,* 2004, vol. 337, 307
- **C.-K. PENG ; J.E. MIETUS ; Y. LIU ; C.LEE ; J.M. HAUSDORFF ; H.E .STANLEY ; A.L. GOLDBERGER ; L.A. LIPSITZ.** Quantifying Fractal Dynamics of Human Respiration: Age and Gender Effects. *Ann. Biomedical Engeneering,* 2002, vol. 30 (5), 683
- **S. THURNER ; S.B. LOWEN ; M.C. FEURSTEIN ; C. HENEGHAN ; H.G. FEICHTINGER ; M.C. TEICH.** Analysis, synthesis, and estimation of fractal-rate stochastic point processes. *Fractals,* 1997, vol. 5, 565

- **R.G. TURCOTT ; M.C. TEICH.** Fractal character of the electrocardiogram: distinguishing heart-failure and normal patients. *Ann. Biomed. Eng.,* 1996, vol. 24, 269
- **G.M. VISWANATHAN ; C.-K. PENK ; H.E. STANLEY ; A.L. GOLDBERGER.** Deviations from uniform power law scaling in nonstationary time series. *Phys. Rev. E,* 1997, vol. 55, 845
- **M.C. TEICH ; C. HENEHAN ; S.B. LOWEN ; T. OZAKI ; E. KAPLAN.** Fractal character of the neural spike train in the visual system in the cat. *J. Opt. Soc. Am.,* 1997, vol. 14, 529
- **S.B. LOWEN ; M.C. TEICH.** Auditory-nerve action potentials form a nonrenewal point process over short as well as long time scales. *J. Acoust. Soc. Am.,* 1992, vol. 92, 803
- **Z. CHEN ; P.CH. IVANOV ; K. HU ; H.E. STANLEY.** Effect of nonstationarities on detrended fluctuation analysis. *Physical Review E,* 2002, vol. 65, 041107
- **J.C. ECHEVERRIA ; B.R. HAYES-GILL ; J.A. GROVE ; M.S. WOOLFSON ; G.D.H. CROAKER.** Detrended fluctuation analysis: a suitable method for studying fetal heart rate variability?. *Physiological Measurement,* 2003, vol. 25, 763-774
- **P.S. WILSON ; A.C. TOMSETT ; R. TOUMI.** Long-memory analysis of time series with missing values. *Physical Review E,* vol. 68, 017103
- **J. THEILER ; S. EUBANK ; A. LONGTIN ; B. GALDRIKIAN ; J.D. FARMER.** Testing for nonlinearity in time series: the method of surrogate data. *Physica D,* 1992, vol. 58, 77
- **A.A. VENDRIG ; R. LOUSBERG.** Within-person relationship among pain intensity, mood and physical activity in chronic pain: a naturalistic approach. *Pain,* 1997, vol. 73 (1), 71-76
- **A.L. BARABÁSI.** The origin of burst and heavy tails in human dynamics. *Nature,* 2005, vol. 435, 207
- **R.V.R. PANDYA.** Generalized model for human dynamics. *e-print physics/0512086,* 2005
- **A. VÁZQUEZ ; J.G. OLIVEIRA ; Z. DEZSÖ ; K.-II GOH ; I. KONDOR ; A.L BARABÁSI.** Modelling bursts and heavy tail in human dynamics. *Physical Rev. E,* 2006, vol. 73, 036127
- **B. BASSINGTHWAIGHTE ; L. LIEBOVITCH ; B.J. WEST.** Fractal Physiology. Oxford University Press, 1994
- **A.L. GOLDBERGER ; L.A.N. AMARAL ; J.M. HAUSDORFF ; P.CH. IVANOV ; C.-K. PENG ; H.E. STANLEY.** Fractal dynamics in physiology: Alterations with disease and aging. *PNAS,* 2002, vol. 99 (1), 2466